# EUROPÄISCHE PATENTANMELDUNG

(11) **EP 1 514 678 A1**
(43) Veröffentlichungstag der Anmeldung: **16.03.2005**
(21) Anmeldenummer: 04019131.4
(22) Anmeldetag: 12.08.2004
(51) Int. Cl.: B32B 7/12, B32B 27/18, B32B 27/12, A61F 13/15

(54) **Mehrlagige Vliesanordnung**

(30) Priorität: 10.09.2003 DE 10342123
(71) Anmelder: Innovatec Microfibre Technology GmbH & Co.KG, 53840 Troisdorf (DE)
(72) Erfinder:
(74) Vertreter: Böck, Bernhard

(57) **Zusammenfassung**

Die Erfindung betrifft eine Vliesanordnung, insbesondere für absorptive Hygieneartikel. Die Vliesanordnung ist als mehrlagiger Verbund aufgebaut und umfasst zumindest eine Trägerlage (9) und eine Decklage (3,4). Erfindungsgemäß weist die Vliesanordnung einen aus Heißschmelzkleber (10) bestehenden Film auf. Dabei dient dieser Hotmelt-Film (10) entweder als Träger für bereichsweise auf der Oberfläche des Heißschmelzklebers (10) angeordnetes Superabsorberpolymer (8), oder der Hotmelt-Film (10) bildet selbst die Trägerlage der Vliesanordnung. Ferner betrifft die Erfindung ein Herstellungsverfahren für eine derartige mehrlagige Vliesanordnung.

## Beschreibung

Die Erfindung betrifft eine mehrlagige Vliesanordnung, insbesondere für absorptive Hygieneartikel, nach dem Oberbegriff des Patentanspruchs 1 bzw. dem Oberbegriff des Patentanspruchs 4. Die Erfindung betrifft ferner ein Verfahren zur Herstellung einer Vliesanordnung nach Patentanspruch 24 bzw. Patentanspruch 25.

Vliesanordnungen der eingangs genannten Art kommen beispielsweise, jedoch keineswegs ausschließlich, für Hygieneartikel, wie beispielsweise für Slipeinlagen, Windeln oder Inkontinenzeinlagen, zum Einsatz. Solche Vliesanordnungen bestehen dabei aus mehreren Lagen funktioneller Gewebe bzw. Vliese und besitzen im allgemein die Aufgabe, bei möglichst geringem Eigengewicht und bei möglichst geringer Dicke eine möglichst große Flüssigkeitsmenge aufnehmen zu können. Dabei soll gleichzeitig gewährleistet sein, dass auch bei hoher Flüssigkeitsaufnahme kein Wiederaustritt der Flüssigkeit erfolgt, möglichst auch dann nicht, wenn Druck auf die Vliesanordnung ausgeübt wird.

Die üblicherweise als Absorptionsschicht in gattungsgemäßen Vliesanordnungen eingesetzten Faservliese wie beispielsweise hydrophile Airlaid-Vliese, benötigen Flächengewichte bis über 200g/m² um die geforderte Aufnahme- und Speicherfähigkeit für Flüssigkeiten zu erfüllen. Dies führt zu relativ dicken Hygieneprodukten, die einen verminderten Tragekomfort aufweisen.

Aus dem Stand der Technik ist es ferner bekannt, in gattungsgemäße Vliesanordnungen sogenannte Superabsorberpolymere, wie beispielsweise vernetzte Polyelektrolyte bzw. gelbildende Polyacrylsäureester einzubetten. Aufgrund ihrer extremen Quellfähigkeit bei Flüssigkeitskontakt sind die Superabsorberpolymere besonders geeignet, die im Hygienebereich bestehenden hohen Anforderungen an die spezifische Flüssigkeitsaufnahme zu erfüllen.

Bei den aus dem Stand der Technik bekannten Produkten wird das Superabsorberpolymer jedoch zumeist in eine Schicht bzw. Lage der Vliesanordnung eingebettet, was im allgemeinen durch Einblasen von Superabsorberpolymer-Pulver in das Grundmaterial der entsprechenden Vliesschicht erfolgt. Damit ist jedoch oft eine unzureichende Verankerung der Superabsorberpolymer-Partikel in der Vliesschicht und damit unerwünschtes Ausstauben von Superabsorberpolymer-Partikeln aus der Vliesschicht verbunden.

In der Druckschrift WO 00/39379 A2 ist zur Lösung dieses Problems vorgeschlagen worden, durch Einblasen und Anlagern eines erhitzten SAP/NaHCO₃-Gemisches in den Meltblownfaserprozess für eine bessere Verankerung der Superabsorberpolymer-Partikel im Meltblown-Vlies zu sorgen. Nachteilig ist dabei jedoch die aufwändige technische Realisierung sowie die zu starke Anbindung der Superabsorberpolymer-Partikel an die Vliesfasern, was die freie Oberfläche der Partikel reduziert und damit die Absorptionsfähigkeit des Superabsorberpolymers vermindert.

Neben der geforderten hohen Flüssigkeitsaufnahme, die beispielsweise mittels der beschriebenen Superabsorberpolymere realisiert werden kann, ist es bei den genannten Hygieneartikeln jedoch auch erwünscht, dass die Hygieneartikel wegen des direkten Hautkontakts hautfreundlich sind und einen hohen Tragekomfort aufweisen. Auch geht der Trend inzwischen zu wasserdampfoffenen, d.h. atmungsaktiven Produkten, mit denen der Tragekomfort wesentlich verbessert werden kann. Andererseits sollten derartige Hygieneprodukte auch bei Feuchtigkeitsaufnahme sich nicht nur keinesfalls nass anfühlen, sondern es soll auch - trotz der gewünschten Atmungsaktivität - möglichst kein Feuchtigkeitsaustritt beispielsweise an den Rändern und insbesondere auf der Rückseite des Produkts erfolgen.

Zur Erzielung der Wasserundurchlässigkeit auf der Rückseite gattungsgemäßer Hygieneprodukte kommen bei den aus dem Stand der Technik bekannten Vliesanordnungen häufig PE-Filme zum Einsatz, die teilweise mikroporös ausgestaltet sind, um eine gewisse Atmungsaktivität zu gewährleisten. Durch dieses folienartige Material wird jedoch der gewünschte textile Charakter, bzw. der wünschenswerte weiche Griff an der Materialoberfläche deutlich verschlechtert. Diese nachteilige Eigenschaft weisen insbesondere die aus dem Stand der Technik bekannten mikroporösen PE-Filme auf, welche zur Erreichung der Wasserdampfdurchlässigkeit oft mit hohen Füllstoffanteilen, beispielsweise mit CaCO₃ angereichert sind.

Mit diesem Hintergrund ist es die Aufgabe der vorliegenden Erfindung, eine Vliesanordnung bzw. ein Verfahren zur Herstellung einer Vliesanordnung zu schaffen, womit die genannten Nachteile überwunden werden. Insbesondere soll sich die Vliesanordnung für kostengünstig zu fertigende Hygieneprodukte mit hohem Tragekomfort, hoher Saugfähigkeit und hoher Auslaufsicherheit eignen.

Diese Aufgabe wird durch eine Vliesanordnung nach der Lehre des Patentanspruchs 1 bzw. nach der Lehre des Patentanspruchs 4 und durch ein Herstellungsverfahren für eine Vliesanordnung mit den Merkmalen des Patentanspruchs 24 bzw. mit den Merkmalen des Patentanspruchs 25 gelöst.

Bevorzugte Ausführungsformen sind Gegenstand der Unteransprüche.

Die Vliesanordnung gemäß der vorliegenden Erfindung ist in zunächst an sich bekannter Weise als mehrlagiger Verbund aufgebaut, wobei der mehrlagige Verbund zumindest eine Trägerlage und eine Decklage umfasst.

Erfindungsgemäß umfasst die Vliesanordnung jedoch zunächst einen aus Heißschmelzkleber (Hotmelt) bestehenden Film. Dabei dient dieser Hotmelt-Film entweder als Träger für partikelförmiges Superabsorberpolymer, wobei das Superabsorberpolymer selektiv bzw. bereichsweise auf der Oberfläche des Heißschmelzklebers angeordnet ist, oder der Hotmelt-Film bildet selbst bereits die Trägerlage der Vliesanordnung.

Dank der Abdichtungswirkung des unmittelbar auf die Trägerlage aufgebrachten und selektiv mit Superabsorberpolymer beschichteten, bzw. selbst die Trägerlage bildenden Heißschmelz-Klebefilms kann auf die nach dem Stand der Technik oftmals verwendete PE-Folie auf der Rückseite der Vliesanordnung verzichtet werden, ohne dass dadurch Funktionseinbußen bezüglich Flüssigkeitsaufnahme oder Flüssigkeitsdichtigkeit der Vliesanordnung gemacht werden müssten.

Da der Heißschmelz-Klebefilm auch äußerst dünn gehalten werden kann, werden bei Verwendung eines Trägervlieses die mechanischen Eigenschaften sowie der wünschenswerte weiche Griff an der Materialoberfläche des Trägervlieses praktisch nicht beeinflusst oder verändert. Dies kommt einerseits einer optimalen Verarbeitungsfähigkeit der Vliesanordnung bei der Herstellung von Hygieneartikeln entgegen, und verbessert andererseits den Tragekomfort derartiger Hygieneartikel erheblich. Zudem erfolgt der Auftrag des Heißschmelzklebers auf das Trägervlies im heißen Zustand, so dass auf diese Weise eine flächige, innige Verbindung zwischen dem Heißschmelz-Klebefilm und dem Trägervlies erzielt wird.

Beim Trägervlies handelt es sich in diesem Fall bevorzugt um Spinn-, Nadel-, oder Mikrofaservliese (Meltblown) bzw. wasservernadelte Vliese aus Polypropylen, Polyäthylenterephtalat oder Polyäthylen, besonders bevorzugt um Spinnvliese aus Polypropylen bzw. niedrig- oder hochdichtem Polyäthylen. Das Flächengewicht des Trägervlieses liegt bei der Verwendung eines solchen Spinnvlieses bevorzugt im Bereich von 8 bis 80 g/m². Auch diese geringen Flächengewichte tragen zum gewünschten möglichst dünnen Endprodukt bei und ermöglichen im Gegensatz zum Stand der Technik besonders weiche, grifffreundliche Hygieneprodukte mit hohem Tragekomfort.

Wird der Heißschmelz-Klebefilm ohne zusätzliche Trägerlage verarbeitet - bildet der Heißschmelz-Klebefilm mit anderen Worten selbst die Trägerlage - so führt dies aufgrund völligen Wegfalls von Trägervliesen o. dergl. insbesondere zu äußerst günstigen Herstellungskosten für die Vliesanordnung, ohne dass hierdurch Flüssigkeitsaufnahme oder Flüssigkeitsdichtigkeit der Vliesanordnung beeinträchtigt würden. Dies gilt insbesondere auch dann, wenn die Vliesanordnung Superabsorberpolymer enthält, also eine besonders hohe Flüssigkeitsaufnahme ermöglicht.

Wird für den Heißschmelzkleber, wie dies gemäß einer Ausführungsform der Erfindung vorgesehen ist, sog. Kontakt-Hotmelt verwendet, so kann die Heißschmelzkleber-Schicht sogar noch die weitere Aufgabe übernehmen, beim Tragen des Hygieneprodukts durch Anhaften an der Innenseite der Kleidung das Verrutschen des Hygieneprodukts zu verhindern.

Eine Anordnung von Superabsorberpolymer-Partikeln auf der Oberfläche des Heißschmelzklebers ermöglicht einerseits eine äußerst sichere Einbindung der Superabsorberpolymer-Partikel, so dass das Problem des unerwünschten Ausstaubens von Superabsorberpolymer-Partikeln eliminiert wird. Gleichzeitig steht die Oberfläche der so auf dem Heißschmelz-Klebefilm haftenden Superabsorberpolymer-Partikel im Unterschied zum Stand der Technik fast vollständig für die Absorption von Flüssigkeit zur Verfügung. Dabei liegt ein besonderer Vorteil der Einbettung bzw. Anhaftung des SAP-Pulvers am Heißschmelzkleber darin, dass die Vliesanordnung auch nach der Aufnahme großer Mengen von Flüssigkeiten in sich stabil bleibt und sogar bei Verletzungen einer Deckschicht praktisch kein Austritt von SAP-Gel erfolgt.

Zusätzlich jedoch ermöglicht eine bereichsweise, selektive Anordnung der Superabsorberpolymer-Partikel auf dem Heißschmelz-Klebefilm eine äußerst vorteilhafte Steuerung der Flüssigkeitsaufnahme bei Hygieneprodukten, die aus der Vliesanordnung hergestellt sind. Denn die Superabsorberpolymer-Partikel lassen sich auf der Oberfläche des Heißschmelz-Klebefilms beispielsweise so anordnen, dass die größte Menge an Superabsorberpolymer in denjenigen Bereichen des Hygieneartikels zu liegen kommt, in denen erfahrungsgemäß die größte Menge an Flüssigkeit anfällt und aufzunehmen ist.

Ausgangsmaterial und Materialeigenschaften von Heißschmelzkleber bzw. Trägervlies sind dabei zunächst einmal unerheblich für die Verwirklichung der Erfindung, solange sich die gewünschte Verankerungswirkung für die Superabsorberpolymer-Partikel bzw. der Ersatz der Trägerlage durch den Hotmelt-Film erreichen lässt. Gemäß einer bevorzugten Ausführungsform der Erfindung sind jedoch die für die Trägerlage und/oder für den Heißschmelzkleber verwendeten Materialien hydrophob.

Dies führt dazu, dass das Austreten von Flüssigkeit auf der Rückseite von Hygieneprodukten, die aus der Vliesanordnung hergestellt sind, zusätzlich erheblich erschwert oder unterbunden wird. Denn einerseits bildet der Heißschmelz-Klebefilm unabhängig vom für den Heißschmelzkleber verwendeten Material bereits eine wirksame Flüssigkeitsbarriere, ein Verhalten, das andererseits durch die Verwendung eines hydrophoben Materials für den Heißschmelzkleber und/oder für die Trägerlage noch verstärkt wird. Bei besonders hohe Anforderungen an die Dichtigkeit von Hygieneprodukten kann, wie dies eine weitere Ausführungsform der Erfindung vorsieht, für den Heißschmelzkleber ein Material verwendet werden, das dampfdicht ist.

Versuche haben gezeigt, dass eine Vliesanordnung mit besonders vorteilhaften Eigenschaften dann erhalten wird, wenn als Material für den Heißschmelz-Klebefilm Äthylvinylacetate, Polyamide, Polyvinylalkohole oder Polyurethane eingesetzt werden, wie dies auch eine weitere Ausführungsform der Erfindung vorsieht.

Hygieneprodukte mit besonders guten Tragekomfort lassen sich aus der Vliesanordnung dann herstellen, wie dies gemäß einer weiteren Ausführungsform der Erfindung vorgesehen ist, wenn der Heißschmelz-Klebefilm diffusionsoffen, d.h. wasserdampfdurchlässig ist. Auf diese Weise wird nach der Beschichtung des Trägermaterials mit dem Heißschmelz-Klebefilm eine Materialbahn erhalten, die zwar einerseits zuverlässig das Ein- bzw. Durchdringen von Flüssigkeiten und Nässe verhindert, andererseits jedoch den Wegtransport der dampfförmigen Körperfeuchte und deren Abgabe an die Umgebungsluft ermöglicht.

Es hat sich gezeigt, wie dies auch gemäß einer weiteren Ausführungsform der Erfindung vorgesehen ist, dass ein diffusionsoffener Heißschmelz-Klebefilm insbesondere dann erhalten wird, wenn das Material für den Heißschmelz-Klebefilm hydrophile Bestandteile und eine von den hydrophilen Bestandteilen herrührenden Oberflächenspannung von ≤ 20mN/m aufweist.

Die hydrophilen Bestandteile sorgen dafür, dass der Heißschmelz-Klebefilm die gewünschten Durchlasseigenschaften für Wassermoleküle, d.h. für Wasserdampf erhält, wobei jedoch gleichzeitig die Dichtigkeit gegenüber Flüssigkeiten erhalten bleibt. Dabei hat es sich erwiesen, dass sich besonders vorteilhafte Eigenschaften einer Vliesanordnung insbesondere dann einstellen, wenn für die hydrophilen Bestandteile des Heißschmelz-Klebefilms Elastomere auf Basis von Polytetramethylenoxid, Comonomere von Polypropylenoxid und Polyehtylenoxid oder hydrophile Polymerweichmacher verwendet werden. Vor allem letztere zeichnen sich dadurch aus, dass sie besonders kostengünstig sind.

Ein weiterer Vorteil der Erfindung liegt darin, dass sich bereits durch das Prinzip der Ausbildung einer flüssigkeitsdichten Schicht in Form von Hotmelt flüssigkeitsdichte Lagen von fast beliebiger Stärke erzeugen lassen. Bevorzugt jedoch liegt das Auftragsgewicht der Heißschmelzkleberbeschichtung je nach Anwendungsfall zwischen 5 bis 50 g/m². Der auf diese Weise erzeugte Heißschmelz-Klebefilm ermöglicht die gewünschte Feuchtigkeitssperre, ist jedoch gleichzeitig so dünn, dass bei Verwendung eines Trägervlieses der weiche Griff an der Materialoberfläche des Trägervlieses praktisch nicht beeinträchtigt wird. Zudem ist der so erzeugte Heißschmelz-Klebefilm sogar nicht unerheblich dünner als die gemäß Stand der Technik eingesetzten PE-Filme, was nicht nur die Grifffreundlichkeit der Hygieneprodukte verbessert, sondern auch zum wünschenswerten geringen Flächengewicht der Vliesanordnung beiträgt.

Ein weiterer besonderer Vorteil der durch einen Heißschmelz-Klebefilm gebildeten Lage der Vliesanordnung liegt darin, dass der Heißschmelz-Klebefilm bei Verwendung eines Trägervlieses nicht nur einen innigen Verbund mit dem Trägervlies eingeht, sondern darüber hinaus auch noch zur Aufgabe der Kaschierung bzw. Verklebung mit der Decklage herangezogen werden kann. Letzteres gilt auch dann, wenn der Hotmeltfilm die Aufgabe der Trägerschicht mit übernimmt.

Die Decklage umfasst dabei gemäß einer bevorzugten Ausführungsform der Erfindung ein hydrophiles Mikrofaservlies, was der raschen und gründlichen Feuchtigkeitsaufnahme durch die Decklage hindurch entgegenkommt. Denn das so unmittelbar an die Erfassungsschicht, Absorptionsschicht oder an das Superabsorberpolymer der Vliesanordnung angrenzende hydrophile Mikrofaservlies sorgt beim Flüssigkeitseintritt aufgrund seiner starken Dochtwirkung für eine schnelle und großflächige Verteilung der aufgenommenen Flüssigkeit, so dass bei Flüssigkeitseintritt ein möglichst großer Flächenbereich der Erfassungsschicht, Absorptionsschicht oder des Superabsorberpolymers der Vliesanordnung benetzt bzw. aktiviert wird.

Gemäß einer weiteren bevorzugten Ausführungsform der Erfindung besteht die Decklage aus einem sogenannten SM-Verbund, der eine Lage hydrophiles Spinnvlies, hergestellt beispielsweise aus Polypropylen oder Polyäthylen, mit einem bevorzugten Flächengewicht von 8 bis 80 g/m², und eine Lage hydrophiles Meltblown, beispielsweise aus Polypropylen, mit einem bevorzugten Flächengewicht von 10 bis 100 g/m² umfasst. Dabei übernimmt das Meltblown wiederum die Aufgabe der raschen und großflächigen Verteilung und ggf. auch Speicherung eindringender Flüssigkeit. Die mit dem Meltblown verbundene Spinnvlies-Decklage erfüllt hier insbesondere mechanische und dekorative Aufgaben und eignet sich außerdem besonders gut für den direkten Hautkontakt.

Gemäß einer weiteren Ausführungsform der Erfindung ist darüber hinaus das Deckvlies bzw. die Oberfläche einer auf dem Heißschmelz-Klebefilm angeordneten mehrschichtigen Decklage zudem durch öl- wachs- oder fetthaltige Avivagen besonders hautfreundlich ausgestattet. Dies ist bei dem dort vorhandenen direkten Hautkontakt insbesondere für Hygieneartikel wie beispielsweise, jedoch keineswegs ausschließlich, für Slipeinlagen vorteilhaft.

Nach einer weiteren bevorzugten Ausführungsform der Erfindung sind zunächst überstehende Ränder der Trägerlage oder der Decklage der Vliesanordnung um die Kanten der Vliesanordnung umgeschlagen und bilden so zumindest bereichsweise einen Randumschlag um die Außenkanten eines aus der Vliesanordnung hergestellten Hygieneprodukts. Auf diese Weise wird die Vliesanordnung des Hygieneprodukts einerseits mechanisch stabilisiert und die einzelnen Lagen der Vliesanordnung werden zusätzlich aneinander fixiert bzw. miteinander verbunden, was die Handhabung des aus der Vliesanordnung hergestellten Produkts erleichtert und verbessert.

Insbesondere bei Umschlag der Trägerlage wird auf diese Weise das Ausdringen von Flüssigkeit durch die hydrophobe Trägerlage auch an den Rändern des Hygieneprodukts wirksam verhindert. Dies gilt insbesondere im Unterschied zum Stand der Technik, bei dem stattdessen häufig die hydrophile Decklage um die Ränder des Hygieneprodukts umgeschlagen wird, was zwar ebenfalls die Handhabung des Produkts verbessert, jedoch für sich genommen keinen wesentlichen Beitrag zur Sicherheit gegen Flüssigkeitsaustritt leistet. Zusammen mit dem selektiv aufgebrachten Superabsorberpolymer bildet bei der vorliegenden Erfindung jedoch auch ein Umschlagen der Decklage einen wirkungsvollen Schutz gegen Auslaufen. Dies liegt darin begründet, dass die umgeschlagene Decklage hierbei insbesondere die auch für den Auslaufschutz wichtige Aufgabe übernimmt, die einzelnen Lagen der Vliesanordnung sicher zusammenzuhalten, während das seitliche Auslaufen dadurch unterbunden wird, dass in den Randbereichen der Vliesanordnung gar kein Superabsorberpolymer vorhanden ist.

Gemäß einer weiteren Ausführungsform der Erfindung wird für das Superabsorberpolymer zumindest anteilig ein Naturprodukt, insbesondere aus der gemahlenen Frucht des Johannisbrotbaumes verwendet. Aus den Kernen der Frucht des Johannisbrotbaumes lässt sich ein anderweitig bereits vielfach technologisch genutztes Verdickungsmittel, das Carubin extrahieren. Es enthält Polysaccharide, Eiweißstoffe sowie Flavonoide. Dabei ist Carubin fünf Mal so quellfähig wie Stärke und kommt bereits als Bindemittel und Stabilisator in einer Vielzahl von Lebensmittelprodukten zum Einsatz. Die extrem hohe Quellfähigkeit prädestiniert das Carubin auch für den Einsatz als superabsorptionsfähiges Material in einer Vliesanordnung.

Nach weiteren, ebenfalls bevorzugten Ausführungsformen der Erfindung ist dem Superabsorberpolymer Aktivkohle und/oder verkokter Bambus in pulverisierter Form zugesetzt. Auf diese Weise lässt sich vor allem die Geruchsabsorption der Vliesanordnung verbessern, beispielsweise für den Fall, dass Feuchtigkeit für mehrere Stunden dauernde Zeiträume in der Vliesanordnung eingelagert ist. Die Geruchsabsorption einer Vliesanordnung gemäß dieser Ausführungsform gründet sich dabei auf die besonders gute chemische Adsorptionsfähigkeit von derartigem, hochporösem und weitgehend reinem Kohlenstoff, insbesondere in Bezug auf unerwünschte Farb-, Geschmacks- und Geruchsstoffe.

Ferner betrifft die Erfindung ein Verfahren zur Herstellung einer absorptionsfähigen Vliesanordnung. Das erfindungsgemäße Verfahren gemäß der Lehre von Patentanspruch 24 bzw. 25 umfasst dabei die nachfolgend beschriebenen Verfahrensschritte.

Zunächst wird in einem ersten Verfahrensschritt ein Hotmeltfilm auf eine Trägerlage aufgebracht. Anschließend wird in einem weiteren Verfahrensschritt Superabsorberpolymer in Pulverform unmittelbar auf den noch schmelzklebrigen Hotmeltfilm selektiv bzw. nur in bestimmten Flächenbereichen des Hotmeltfilms aufgebracht.

In einem weiteren Verfahrensschritt wird sodann eine Decklage auf Hotmeltfilm und Superabsorberpolymer-Pulver aufgebracht. Schließlich erfolgen in einem weiteren Verfahrensschritt ein Zuschnitt der Vliesanordnung sowie die Verbindung der verschiedenen Lagen der Vliesanordnung miteinander.

Mit dem erfindungsgemäßen Verfahren lassen sich hochabsorptionsfähige Vliesanordnungen mit besonders vorteilhaften Nutzeigenschaften kostengünstig fertigen. Dies hängt zunächst einmal damit zusammen, dass der Hotmeltfilm bei dem erfindungsgemäßen Verfahren mehrere verschiedene Funktionen gleichzeitig erfüllt.

So bildet der Hotmeltfilm einerseits eine Barriere für Flüssigkeiten und ermöglicht so, für die Trägerschicht anstelle der im Stand der Technik häufig eingesetzten Kunststofffolien die besonders hautfreundlichen und griffsympathischen Mikrofaservliese zu verwenden.

Weiterhin dient der Hotmeltfilm auch der Verankerung der aufgebrachten Superabsorberpolymer-Partikel. Dies führt im Vergleich zum eingangs beschriebenen Stand der Technik zu mechanisch besonders stabilen und belastbaren Vliesanordnungen, die nicht zum Ausstauben von Superabsorberpolymer-Pulver neigen und auch keine Tendenz zum Auslaufen nach der Absorption großer Mengen von Flüssigkeiten aufweisen. Schließlich kann der Hotmeltfilm auch noch die Aufgabe der Verklebung der einzelnen Materiallagen untereinander erfüllen, wobei durch das Eindringen des Heißschmelzklebers zwischen die an der Oberfläche der einzelne Materiallagen gelegenen Fasern sogar noch eine weitere Stabilisierung des Verbundes erzielt werden kann.

Überdies lassen sich mit dem erfindungsgemäßen Verfahren Vliesanordnungen mit besonders vorteilhaften Nutzeigenschaften für den Einsatz in Hygieneprodukten herstellen. Dies hängt mit dem selektiven Aufbringen des Superabsorberpolymer-Pulvers auf den noch schmelzflüssigen bzw. schmelzklebrigen Hotmeltfilm zusammen. Denn auf diese Weise kann der größte Teil des Superabsorberpolymer-Pulvers auf diejenigen Flächenbereiche der Vliesanordnung aufgebracht werden, in denen erfahrungsgemäß die größte Flüssigkeitsmenge anfällt bzw. absorbiert werden muss.

Außerdem können auf diese Weise beispielsweise die Randbereiche eines Hygieneprodukts weitgehend frei vom Auftrag von Superabsorberpolymer-Pulver gehalten werden, so dass in diesen Bereichen auch keine Speicherung von nennenswerten Flüssigkeitsmengen erfolgt. Vielmehr wird die anfallende Flüssigkeit damit in die Mitte des Absorptionsbereichs geleitet und dort gespeichert. Diese Gestaltung trägt zusätzlich zur Auslaufsicherheit des Produkts bei. Da auf diese Weise etwa überstehende Randbereiche des Hygieneprodukts ebenfalls kein Superabsorberpolymer enthalten, kann das Material dieser Randbereiche nach dem Abschneiden im Gegensatz zum Stand der Technik vollkommen problemlos recycelt werden.

Ein weiteres erfindungsgemäßes Verfahren zur Herstellung einer absorptionsfähigen Vliesanordnung umfasst in einem ersten Verfahrensschritt das Aufbringen von zumindest einer Lage Hotmeltfilm auf ein Trennpapier. In einem weiteren Verfahrensschritt wird sodann eine Decklage auf den Hotmeltfilm aufgebracht. Schließlich erfolgt in einem weiteren Verfahrensschritt Zuschnitt und Verbindung der Lagen der Vliesanordnung untereinander.

Dieses Herstellungsverfahren besitzt insbesondere den Vorteil, dass sich hochwertige absorptionsfähige Vliesanordnungen damit äußerst kostengünstig fertigen lassen, da der Hotmeltfilm hier auch noch die Aufgabe der Trägerschicht unter Wegfall ansonsten zusätzlich notwendiger Schichten übernimmt. Wird zudem für den Hotmeltfilm ein Kontakt-Hotmelt verwendet, so kann ein und derselbe Hotmeltfilm zudem auch noch die Aufgabe der Haftstreifen übernehmen, die am fertigen Hygieneprodukt das Verrutschen des Hygieneprodukts durch Anhaften an der Innenseite der Kleidung verhindern.

Eine solche Vliesanordnung mit einem Hotmeltfilm als Trägerschicht kann als Bestandteil einer weiteren Lage, oder aber als unmittelbar auf den noch schmelzklebrigen Hotmeltfilm aufgebrachte Auflage Superabsorberpolymer enthalten, um die Absorptionseigenschaften eines daraus hergestellten Hygieneprodukts zu verbessern. Das Superabsorberpolymer ist dabei bevorzugt partikelförmig und wird besonders bevorzugt selektiv lediglich bereichsweise auf dem Hotmeltfilm angeordnet.

Dabei ist es für die Erfindung zunächst einmal nicht wesentlich, auf welche Weise das selektive Aufbringen der Superabsorberpolymer-Partikel auf die Schmelze des Hotmeltfilms erfolgt, solange sich die vorgesehene ungleichförmige Verteilung der Superabsorberpolymer-Partikel bei den erforderlichen kurzen Taktzeiten bzw. hohen Durchlaufgeschwindigkeiten realisieren lässt.

Gemäß einer besonders bevorzugten Ausführungsform der Erfindung erfolgt jedoch das selektive Aufbringen der Superabsorberpolymer-Partikel durch eine Druckwalze, insbesondere eine Gravurwalze. Diese im Prinzip den Druckverfahren entlehnte Vorgehensweise ist äußerst vorteilhaft insofern, als sich damit fast beliebig hohe Durchlaufgeschwindigkeiten - üblich sind hier Geschwindigkeiten bis nahezu 300 Meter/min - bzw. fast beliebig kurze Taktzeiten erreichen lassen.

Dabei werden die der gewünschten Verteilung des Superabsorberpolymer-Pulvers entsprechenden Vertiefungen der Gravurwalze aus einem Reservoir mit Superabsorberpolymer-Pulver gefüllt, wobei überschüssige Mengen an Pulver von einer Rakel abgestreift und rückgeführt werden. Das in der Gravur verbleibende Superabsorberpolymer-Pulver wird sodann mittelbar oder unmittelbar in der gewünschten Flächenverteilung auf den noch klebrigen Hotmeltfilm übertragen.

Gemäß einer weiteren Ausführungsform der Erfindung erfolgt das selektive Aufbringen von Superabsorberpolymer-Partikeln durch elektrostatisches Sprühen. Auch auf diese Weise lassen sich beliebige Flächenverteilungen des Superabsorberpolymer-Pulvers beim Auftrag auf den Hotmeltfilm erzielen.

Die Erfindung wird verwirklicht unabhängig davon, welche Art von Decklage auf dem Hotmeltfilm angeordnet wird. Im einfachsten Fall kann die Decklage beispielsweise aus einem hydrophilen Faservlies bestehen, das bereits für eine gute Flüssigkeitsaufnahme und Flüssigkeitsverteilung sorgt. Nach einer weiteren bevorzugten Ausführungsform der Erfindung liegt die Decklage jedoch in Form eines hydrophilen SM-Verbundes aus Spinnvlies und Meltblown vor.

Hierdurch lassen sich besonders absorptionsfähige Vliesanordnungen erhalten, da das Meltblown aufgrund seiner starken Kapillar- bzw. Dochtwirkung eingedrungene Flüssigkeiten rasch über eine große Fläche verteilen kann, wodurch rasch ein großer Flächenbereich beispielsweise des Superabsorberpolymer-Pulvers benetzt bzw. aktiviert wird. Das Spinnvlies übernimmt dabei insbesondere die Aufgabe der mechanischen Stabilisierung und Begrenzung des Meltblown an der Materialoberfläche.

Gemäß einer weiteren Ausführungsform der Erfindung werden in einem zusätzlichen Verfahrensschritt überstehend angeordnete Randbereiche der Trägerlage oder der Decklage um die korrespondierenden Randbereiche der anderen Lagen der Vliesanordnung herumgefaltet und bilden so einen Randumschlag des aus der Vliesanordnung hergestellten Hygieneprodukts.

Auf diese Weise lässt sich die das Hygieneprodukt bildende Vliesanordnung einerseits mechanisch stabilisieren und es werden die einzelnen Lagen der Vliesanordnung zusätzlich aneinander fixiert bzw. miteinander verbunden. Dadurch wird die Handhabung des aus der Vliesanordnung hergestellten Produkts verbessert und erleichtert. Andererseits wird insbesondere durch den Umschlag der hydrophoben Trägerlage auf diese Weise das Ausdringen von Flüssigkeit auch an den Rändern des Hygieneprodukts sicher unterbunden.

Für die Erfindung ist es nicht erheblich, auf welche Weise die mechanische Verbindung der Lagen der Vliesanordnung erfolgt. Gemäß einer bevorzugten Ausführungsform erfolgt die Verbindung der Lagen der Vliesanordnung jedoch durch Kalandrieren, was insbesondere den Vorteil hat, in-Line mit voller Bahngeschwindigkeit durchgeführt werden zu können.

Eine weitere Ausführungsform des erfindungsgemäßen Verfahrens sieht vor, dass in weiteren Verfahrensschritten auf der Rückseite der Trägerschicht ein Auftrag von Heißschmelz-Kontaktkleber sowie von Trennpapier, beispielsweise Silikonpapier, erfolgt. Auf diese Weise kann das zuvor hergestellte Hygieneprodukt mit dem zur Fixierung des Hygieneprodukts bei der Anwendung notwendigen Klebestreifen versehen werden, und der Klebestreifen kann gleichzeitig die notwendige Schutzabdeckung erhalten.

Es ist für das Wesen der Erfindung ferner nicht ausschlaggebend, wie die betriebstechnische Umsetzung des erfindungsgemäßen Verfahrens erfolgt. So kann das erfindungsgemäße Verfahren beispielsweise diskontinuierlich bzw. Off-Line durchgeführt werden. Gemäß einer bevorzugten Ausführungsform der Erfindung werden jedoch sämtliche Verfahrensschritte im getakteten bzw. kontinuierlichen Inline-Verfahren durchgeführt. Auf diese Weise lässt sich ein besonders hoher Durchsatz bei gleichzeitig hoher Reproduzierbarkeit der Qualitätsparameter der Vliesanordnung erzielen.

Im Folgenden wird die Erfindung anhand lediglich Ausführungsbeispiele darstellender Zeichnungen näher erläutert.

Es zeigt:
- **Fig. 1**: in nicht maßstabsgetreuer, schematischer seitlicher Schnittdarstellung die prinzipielle Funktionsweise einer Vliesanordnung gemäß dem Stand der Technik;
- **Fig. 2**: in einer **Fig. 1** entsprechenden Darstellung den prinzipiellen Aufbau und die prinzipielle Funktionsweise einer Vliesanordnung gemäß einer Ausführungsform der vorliegenden Erfindung;
- **Fig. 3**: in einer **Fig. 1** und **2** entsprechenden Darstellung prinzi-piellen Aufbau und Funktionsweise einer Vliesanordnung gemäß einer weiteren Ausführungsform der vorliegenden Erfindung;
- **Fig. 4**: in einer **Fig. 1** bis **3** entsprechenden Darstellung den prinzipiellen Aufbau einer Vliesanordnung gemäß einer alternativen Ausführungsform der vorliegenden Erfindung;
- **Fig. 5**: in einer **Fig. 1** bis **4** entsprechenden Darstellung den prinzipiellen Aufbau einer Vliesanordnung gemäß einer weiteren Ausführungsform der vorliegenden Erfindung;
- **Fig. 6**: in einer **Fig. 1** bis **5** entsprechenden Darstellung ein Ausführungsbeispiel einer Vliesanordnung;
- **Fig. 7**: in einer **Fig. 1** bis **6** entsprechenden Darstellung ein Ausführungsbeispiel einer Vliesanordnung mit Meltblown als Decklage;
- **Fig. 8**: in einer **Fig. 1** bis **7** entsprechenden Darstellung ein Ausführungsbeispiel einer Vliesanordnung mit einem Dryblend aus Superabsorberpolymer und Hotmelt;
- **Fig. 9**: in einer **Fig. 1** bis **8** entsprechenden Darstellung ein weiteres Ausführungsbeispiel einer Vliesanordnung mit Meltblown als Decklage und Hotmelt als Trägerlage;
- **Fig. 10**: in einer **Fig. 1** bis **9** entsprechenden Darstellung ein weiteres Ausführungsbeispiel einer Vliesanordnung mit Spunbound als Decklage und Hotmelt als Trägerlage;
- **Fig. 11**: in einer **Fig. 1** bis **10** entsprechenden Darstellung eine alternative Vliesanordnung mit Hotmelt als Trägerlage;
- **Fig. 12**: in einer **Fig. 1** bis **11** entsprechenden Darstellung eine weitere Vliesanordnung mit Hotmelt als Trägerlage;
- **Fig. 13**: in einer **Fig. 1** bis **12** entsprechenden Darstellung eine Vliesanordnung mit Kontakt-Hotmelt als Trägerlage; und
- **Fig. 14**: in einer **Fig. 1** bis **13** entsprechenden Darstellung eine Vliesanordnung zusätzlichem SAP-Pulver innerhalb des Bereichs der Meltblown-Lage.

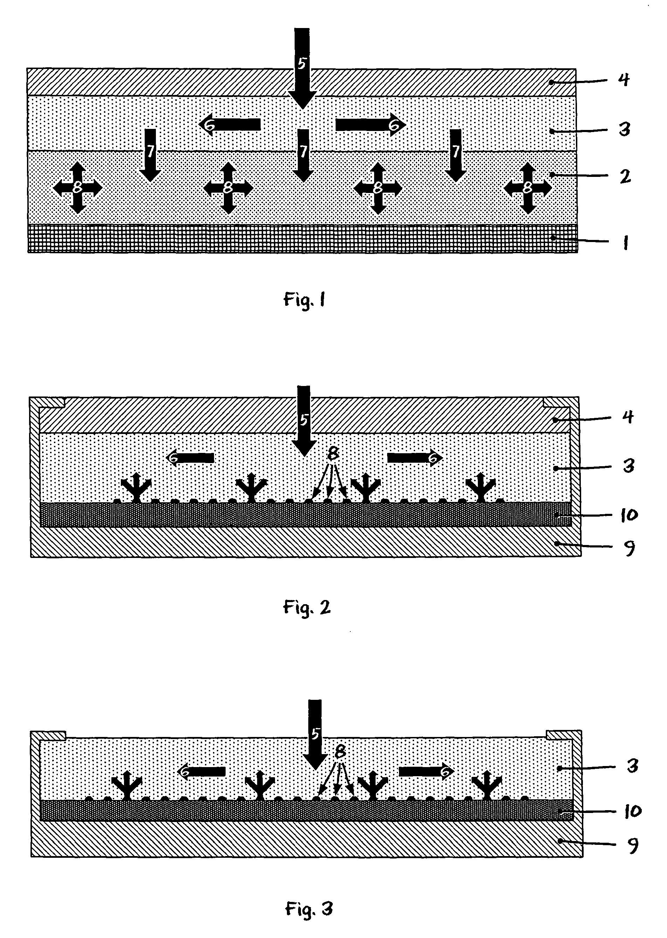

In **Fig. 1** ist in schematischer Darstellung der Querschnitt einer mehrlagigen Vliesanordnung dargestellt, wie sie im Prinzip aus dem Stand der Technik bekannt ist.

Man erkennt zunächst die zeichnungsbezogen unten angeordnete Trägerschicht 1, die gemäß dem Stand der Technik im Allgemeinen aus einer ggf. mikroporösen Polyäthylenfolie besteht. Unmittelbar auf der Trägerschicht 1 ist ein Absorptionskern 2 angeordnet, der aus einem hochabsorptionsfähigen Material, beispielsweise aus einer superabsorberpolymerhaltigen Materiallage besteht. Oberhalb des Absorptionskerns 2 wiederum befindet sich die Erfassungsschicht 3, die der raschen Absorption sowie der Flüssigkeitsverteilung innerhalb der Ebene der Vliesanordnung dient. Die Erfassungsschicht 3 besteht im Allgemeinen aus einem Stapelfaservlies oder Meltblown. Ferner umfasst die Vliesanordnung die Deckschicht 4, die im Allgemeinen in Form eines flüssigkeitsdurchlässigen, hydrophilen Spinnvlieses vorliegt.

Man erkennt in **Fig. 1** außerdem das Prinzip der Flüssigkeitsaufnahme sowie der Flüssigkeitsverteilung und -speicherung in der Vliesanordnung. Der mit Bezugsziffer 5 bezeichnete Pfeil steht für eine auf die Deckschicht 4 aufgebrachte Flüssigkeitsmenge, die aufgrund des offenporigen Charakters der Deckschicht 4 sowie aufgrund der starken Kapillar- bzw. Dochtwirkung der Erfassungsschicht 3 sofort durch die Deckschicht 4 hindurch gesaugt und zunächst von der Erfassungsschicht 3 aufgesogen wird. Ebenso aufgrund der starken Dochtwirkung wird die aufgesogene Flüssigkeitsmenge 5 innerhalb der Fläche der Erfassungsschicht 3 zeichnungsbezogen horizontal verteilt, was die mit Bezugsziffer 6 bezeichneten Pfeile verdeutlichen.

Im Anschluss an die horizontale Verteilung 6 der eingedrungenen Flüssigkeit 5 wird diese von der Erfassungsschicht 3 an die Absorptionsschicht 2 weitergegeben, was die mit Bezugsziffer 7 bezeichneten Pfeile andeuten. Innerhalb der Absorptionsschicht 2 wird die Flüssigkeitsmenge 5 durch Aktivierung, Expansion und Gelbildung des in der Absorptionsschicht 2 enthaltenen Superabsorberpolymers schließlich aufgenommen und als Gel gebunden, was die eine Expansion des Superabsorberpolymers andeutenden Pfeile 8 versinnbildlichen.

Wie eingangs beschrieben, weist dieser aus dem Stand der Technik bekannte Aufbau der Vliesanordnung diverse Nachteile auf, von denen hier insbesondere der mangelnde Tragekomfort solchermaßen aufgebauter Hygieneprodukte bzw. die mangelnde Grifffreundlichkeit der die Trägerschicht darstellenden Polyäthylenfolie 1, der komplizierte Aufbau, der mangelnde Zusammenhalt der einzelnen Schichten und die mangelnde Verankerung des Superabsorberpolymers 8 innerhalb der Absorptionsschicht 2 nochmals genannt werden sollen.

Außerdem nachteilig ist die Neigung der in **Fig. 1** dargestellten Vliesanordnung zum seitlichen Flüssigkeitsaustritt. Dies hängt einerseits damit zusammen, dass bei der in **Fig. 1** dargestellten Vliesanordnung keine seitlichen Barrieremaßnahmen getroffen sind, die den seitlichen Flüssigkeitsaustritt verhindern können. Andererseits hängt die Neigung zum Flüssigkeitsaustritt aber auch damit zusammen, dass sämtliche Lagen der Vliesanordnung gemäß **Fig. 1** einen homogenen Aufbau aufweisen, weshalb zentral eintretende Flüssigkeit 5 auch zu den Seiten der Vliesanordnung geleitet wird, wo sie ggf. austreten kann.

Diese Nachteile werden durch die in **Fig. 2** dargestellte, eine Ausführungsform der vorliegenden Erfindung verkörpernde Vliesanordnung eliminiert. Die Vliesanordnung gemäß **Fig. 2** besitzt ebenfalls eine Trägerschicht 9. Zunächst einmal liegt im Unterschied zum Stand der Technik die Trägerschicht 9 gemäß **Fig. 2** jedoch nun ebenso wie die Deckschicht 4 in Form eines atmungsaktiven Faservlieses vor, was insbesondere bereits den Tragekomfort und den "Look-and-Feel" dergestalt aufgebauter Hygieneprodukte erheblich verbessert.

Ferner wird die erforderliche Flüssigkeitsdichtigkeit bei der Vliesanordnung gemäß **Fig. 2** durch eine unmittelbar auf der Trägerschicht 9 aufgebrachte, flüssigkeitsdichte Membran 10 gewährleistet, die in Form einer Heißschmelzkleber-Schicht 10 vorliegt. Als Ausgangsmaterialien für die Hotmeltschicht 10 haben sich Produkte der Firma Wetzel, Herford, bewährt, die unter den Handelsnamen Pergitex 99932 bzw. Pergitex 23535 bekannt sind. Der Auftrag der Hotmeltschicht 10 erfolgt vorzugsweise mit einer Vorhang-Schlitzdüse, wobei sich hierzu insbesondere Curtain Coating-Düsen der Firma Inatec, Langenfeld als geeignet erwiesen haben.

Unmittelbar auf die Hotmelt-Schicht 10 wiederum ist Superabsorberpolymer 8 in Form von fein verteilten Pulverpartikeln aufgebracht. Damit ist insbesondere der Vorteil verbunden, dass das Superabsorberpolymer 8 über den Hotmeltfilm 10 mit der Trägerschicht 9 verbunden bzw. räumlich verankert ist, was ganz beträchtlich zur mechanischen Stabilität, Auslaufsicherheit und damit Gebrauchstauglichkeit eines so aufgebauten Hygieneprodukts beiträgt.

Zeichnungsbezogen oberhalb des Hotmeltfilms 9 bzw. des Superabsorberpolymers 10 ist wieder eine Erfassungsschicht 3 angeordnet, die der schnellen Aufsaugung und flächigen Verteilung 6 einer auf die Vliesanordnung aufgebrachten Flüssigkeitsmenge 5 dient. Daran anschließend wird die Flüssigkeitsmenge 5 durch Aktivierung, Expansion und Gelbildung der Superabsorberpolymerkügelchen 8 in der Vliesanordnung gemäß **Fig. 2** gebunden, was die eine Expansion des Superabsorberpolymers 8 versinnbildlichenden Dreifachpfeile darstellen.

Zudem ist jedoch das Superabsorberpolymer-Pulver 8 bei der Vliesanordnung gemäß **Fig. 2** lediglich bereichsweise auf die Hotmelt-Schicht 10 aufgebracht, so dass sich im mittleren Bereich der Vliesanordnung eine höhere Konzentration an Superabsorberpolymer 8 als in den Randbereichen ergibt. Dies bedeutet mit anderen Worten, dass das Superabsorberpolymer 8 bei der Vliesanordnung gemäß **Fig. 2** primär in denjenigen Bereichen des Hygieneprodukts angeordnet ist, in denen erfahrungsgemäß die größte Menge an Flüssigkeit 5 anfällt und aufgenommen werden muss. Bereits aus diesem Grund ist auch die seitliche Auslaufneigung eines gemäß **Fig. 2** gestalteten Hygieneprodukts erheblich verringert, da in den Rand- bzw. Kantenbereichen des Hygieneprodukts kaum Flüssigkeit 5 aufgenommen und gespeichert wird. Vielmehr wird die Flüssigkeitsmenge in demjenigen zentralen Bereich des Hygieneprodukts zusammengezogen, in dem zuvor der selektive Auftrag des Superabsorberpolymer-Pulvers 8 erfolgte.

Außerdem führt der selektive Auftrag des Superabsorberpolymer-Pulvers 8 auf den Hotmeltfilm 10 dazu, dass Schnittabfälle des Meltblown-Vlieses 3, die beim Zuschnitt der Vliesanordnung anfallen, problemlos recycelt werden können, da sie kein Superabsorberpolymer-Pulver 8 enthalten.

Die Decklage 3, 4 der Vliesanordnung gemäß **Fig. 2** kann vorzugsweise aus einem sogenannten **SM**-Verbund bestehen, der beispielsweise als ein aus Spinnvlies 4 und Meltblown 3 bestehendes Halbzeug vorliegt. In diesem Zusammenhang kommt ein weiterer Vorteil der Vliesanordnung gemäß **Fig. 2** zum Tragen, der mit der Mehrfachfunktion des Hotmeltfilms 10 zusammenhängt. Denn der Hotmeltfilm 10 dient sowohl der Erzeugung der flüssigkeitsdichten Lage 10 der Vliesanordnung als auch der gegenseitigen Verankerung von Trägerschicht 9, Superabsorberpolymer 8 und SM-Decklage 3, 4. Dadurch ermöglicht diese Verankerung der verschiedenen Lagen der Vliesanordnung untereinander die Herstellung mechanisch besonders belastbarer und damit besonders hochwertiger Hygieneprodukte.

Ein weiterer Vorteil der Vliesanordnung gemäß **Fig. 2** liegt darin, dass die vorzugsweise als hydrophobes Vlies ausgeführte Trägerschicht 9 um die Kanten der Vliesanordnung bzw. um die Kanten eines so aufgebauten Hygieneprodukts herumgeführt ist und sich damit ein zusätzlicher wirksamer Auslaufschutz an den Kanten des Produkts ergibt.

**Fig. 3** zeigt Aufbau und Funktionsweise einer Vliesanordnung gemäß einer weiteren Ausführungsform der Erfindung.

Man erkennt einen Aufbau der Vliesanordnung, der demjenigen der Vliesanordnung gemäß **Fig. 2** weitgehend gleicht. Der Unterschied zu der Vliesanordnung gemäß **Fig. 2** liegt darin, dass bei der Vliesanordnung gemäß **Fig. 3** die Decklage lediglich durch eine einzige Schicht eines hydrophilen Meltblown 3 gebildet ist. Dies dient einerseits der wünschenswerten geringen Gesamtstärke der Vliesanordnung bzw. des dergestalt aufgebauten Hygieneprodukts. Andererseits sind mit der Herstellung der Vliesanordnung gemäß **Fig. 3** weniger Arbeitsschritte verbunden, was zu einer kostengünstigen Herstellbarkeit und einem dementsprechend günstigen Preispunkt eines so aufgebauten Hygieneprodukts führt.

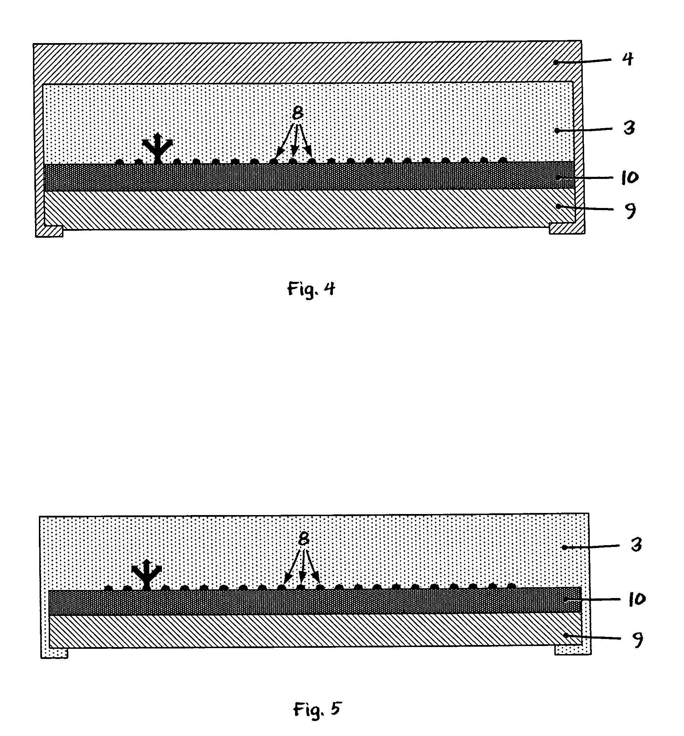

**Fig. 4** zeigt den prinzipiellen Aufbau einer alternativen Ausführungsform der Vliesanordnung gemäß der vorliegenden Erfindung. Man erkennt den Lagenaufbau der Vliesanordnung gemäß **Fig. 4**, der weitestgehend dem Lagenaufbau der Vliesanordnung gemäß **Fig. 2** entspricht. Insbesondere gilt dies, zeichnungsbezogen von unten nach oben betrachtet, für die Abfolge von Trägervlies 9, Hotmeltfilm 10, selektiver Superabsorberpolymerauflage 8 sowie der aus einem SM-Verbund bestehenden Decklage 3, 4. Im Unterschied zu der Vliesanordnung gemäß **Fig. 2** ist bei der Vliesanordnung gemäß **Fig. 4** nicht das Trägervlies 9, sondern die Spunbond-Schicht 4 der SM-Decklage 3, 4 um die Kanten der Vliesanordnung herumgeführt.

Gleiches gilt auch für den Unterschied zwischen der Vliesanordnung gemäß **Fig. 5** und der Vliesanordnung gemäß **Fig. 3**. Auch bei diesen beiden Vliesanordnungen liegt jeweils der gleiche Lagenaufbau vor. Auch die Vliesanordnung gemäß **Fig. 5** verzichtet wie die Vliesanordnung gemäß **Fig. 3** zu Gunsten einer kostengünstigen Herstellbarkeit und einen geringeren Schichtdicke auf eine Decklage 4 aus Spunbound. Vielmehr wird bei der Vliesanordnung gemäß **Fig. 5** ebenso wie bei der Vliesanordnung gemäß **Fig. 3** die Decklage durch die Meltblown-Schicht 3 selbst gebildet. Bei der Vliesanordnung gemäß **Fig. 5** bildet die Meltblown-Schicht 3 ferner auch die seitliche Begrenzung der Vliesanordnung bzw. des Hygieneprodukts, indem die Meltblown-Schicht 3 an den Kanten des Produkts um die weiteren Lagen der Vliesanordnung, nämlich Hotmelt 10 mit Superabsorberpolymer 8 sowie Trägervlies 9, herumgeführt ist.

Obwohl die Spunbound-Lage 4 der SM-Decklage 3, 4 (**Fig. 4**) bzw. die Meltblown-Schicht 3 (**Fig. 5**) jeweils hydrophil, also nicht wie das Trägervlies 9 hydrophob ist, kann bei den Vliesanordnungen gemäß **Fig. 4** und **5** bzw. bei dergestalt aufgebauten Hygieneprodukten dennoch ein guter und wirksamer Auslaufschutz auch an den Kanten des Produkts erzielt werden. Dies hängt insbesondere damit zusammen, dass der Auftrag des Superabsorberpolymers 8 selektiv erfolgt, was in **Fig. 4** und 5 dadurch versinnbildlicht wird, dass Superabsorberpolymer-Partikel 8 nur im mittleren Bereich der Fläche der Vliesanordnung angeordnet sind, nicht jedoch im kantennahen Randbereich der Vliesanordnung bzw. des Hygieneprodukts.

Die Verbesserung des Zusammenhalts sämtlicher Lagen der Vliesanordnung bzw. des Hygieneprodukts durch die seitlich herumgeführte Decklage 4 oder 3 (**Fig. 4** und **5**) bzw. Trägerlage 9 (**Fig. 2** und **3**) ist natürlich unabhängig davon gegeben, ob die hydrophile Decklage 4 bzw. 3 oder die hydrophobe Trägerlage 9 die Schichten der Vliesanordnung umgreift.

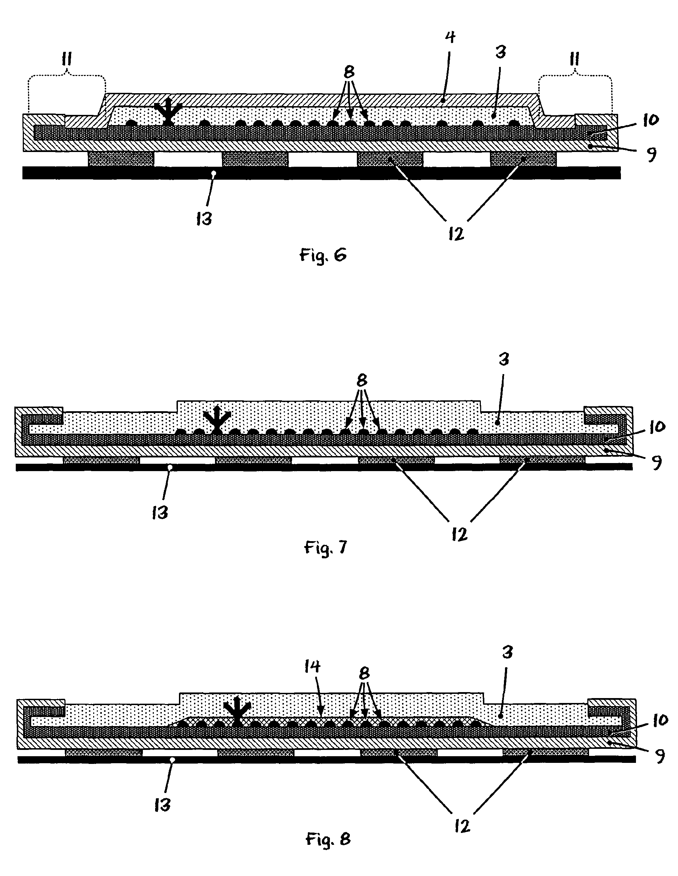

Die **Fig. 6** bis **13** zeigen weitere Ausführungsformen von Vliesanordnungen bzw. Hygieneprodukten in ebenfalls schematischer und nichtmaßstäblicher Darstellung, wobei die Darstellung der **Fig. 6** bis **13** jedoch weniger abstrahierend als die Darstellung der **Fig. 1** bis **5** gewählt wurde und sich daher der tatsächlichen Ausführung jeweiliger Hygieneprodukte annähert.

**Fig. 6** zeigt eine Vliesanordnung bzw. ein Hygieneprodukt, das wiederum Trägervlies 9, Hotmelt-Schicht 10 mit Superabsorberpolymer-Partikeln 8 sowie Meltblown-Erfassungsschicht 3 und Spunbound-Deckschicht 4 umfasst. Dabei ist das Trägervlies 9 um die seitlichen Kanten des Hygieneprodukts herumgeführt. Dieser seitliche Umschlag des Trägervlieses 9 führt zusammen mit dem selektiven Auftrag des Superabsorberpolymers 8 sowie zusammen mit der nicht die ganze Breite des Produkts bedeckenden Meltblown-Erfassungsschicht 3, welche zu einem ebensolchen Einzug 11 der Spunbound-Deckschicht 4 führt, zu einem äußerst wirksamen Auslaufschutz an den seitlichen Kanten des Produkts. Zudem lässt sich auf diese Weise ein Hygieneprodukt erhalten, das aufgrund des innigen Verbunds der Lagen miteinander eine hohe Anwendungsfreundlichkeit und besonders gute Stabilität bei der Handhabung im trockenen wie im feuchten Zustand besitzt.

Zusätzlich ist die Vliesanordnung bzw. das Hygieneprodukt gemäß **Fig. 6** mit einem punkt- oder streifenförmigen Haftschmelzkleberauftrag 12 auf der Rückseite des Trägervlieses versehen. Der dort angeordnete Haftschmelzkleber 12 dient der Fixierung des Hygieneprodukts auf der Innenfläche der Kleidung während des Tragens des Hygieneprodukts. Zum Schutz des Haftschmelzkleberauftrags 12 ist auf der außenliegenden Seite des Haftschmelzklebers 12 noch ein Trennpapier 13, beispielsweise ein Silikonpapier, angeordnet.

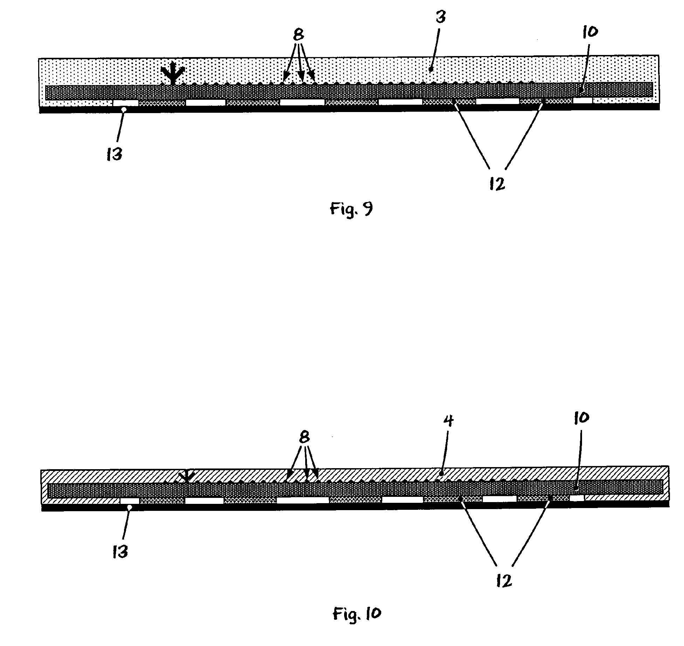

Einen prinzipiell ähnlichen Aufbau wie die Vliesanordnung bzw. das Hygieneprodukt gemäß **Fig. 6** besitzen auch die Vliesanordnungen bzw. Hygieneprodukte gemäß **Fig. 7** und **8**. Der wesentliche Unterschied zwischen den Vliesanordnungen gemäß **Fig. 7** bzw. **8** und der Vliesanordnung gemäß **Fig. 6** ist ähnlich gelagert wie der Unterschied zwischen den Vliesanordnungen gemäß den **Fig. 5** und **4** bzw. der Unterschied zwischen den Vliesanordnungen gemäß in **Fig. 2** und **3**. Dies bedeutet mit anderen Worten, dass auch die Vliesanordnungen gemäß **Fig. 7** und **8** zu Gunsten einer kostengünstigen Herstellbarkeit sowie zu Gunsten geringer Schichtdicken auf eine separate Spunbound-Decklage 4 verzichten, wodurch jeweils die Meltblown-Schicht 3 der Vliesanordnungen gemäß **Fig. 7** und **8** gleichzeitig die Aufgaben von Deckschicht 4 und Erfassungsschicht 3 übernimmt.

Ein weiterer Unterschied zwischen den Vliesanordnungen gemäß **Fig. 7** bzw. **8** und der Vliesanordnung gemäß **Fig. 6** liegt darin, dass nicht nur das Trägervlies 9, sondern auch die auf dem Trägervlies 9 angeordnete Hotmeltschicht 10 um die Außenkanten des Produkts herumgefaltet wurde. Dies führt trotz des Verzichts auf die separate Deckschicht 4 zu einem guten Auslaufschutz an den Kanten der Vliesanordnung.

Der Unterschied zwischen den Vliesanordnungen gemäß **Fig. 8** und **Fig. 7** liegt darin, dass bei der Vliesanordnung gemäß **Fig. 8** auf der Hotmeltschicht 10 nicht lediglich wie in **Fig. 7** ein Auftrag von Superabsorberpolymer-Pulver 8 erfolgte, sondern dass bei der Herstellung der Vliesanordnung gemäß **Fig. 8** auf die Hotmeltschicht 10 ein Dryblend aus Superabsorberpolymer-Pulver 8 und Hotmelt-Pulver 14 aufgetragen wurde. Dies hat sich als besonders vorteilhaft für eine sichere Verfahrensführung des Superabsorberpolymer-Auftrags auf die Hotmeltschicht 10 erwiesen.

Alternative Ausführungsformen von Vliesanordnungen sind in den **Fig. 9** bis **13** dargestellt. Diese zeichnen sich insbesondere dadurch aus, dass auf ein Trägervlies 9 komplett verzichtet wurde, wodurch die Trägerlage nur noch durch den Hotmeltfilm 10 selbst gebildet wird. Auch dies führt zum Wegfall von ansonsten notwendigen Herstellungsverfahrensschritten sowie zum Wegfall des Trägervlieses 9 und reduziert so vorteilhaft sowohl die Herstellungskosten als auch die Dicke des so hergestellten Hygieneprodukts.

Die Ausführungsformen gemäß **Fig. 9** und **10** ähneln in ihrem vereinfachten Aufbau ohne Separierung von Deckschicht 4 und Erfassungsschicht 3 den Ausführungsformen insbesondere gemäß **Fig. 3, 7** oder **8**. Im Unterschied zu den Ausführungsformen gemäß **Fig. 3, 7** oder **8** ist bei den Ausführungsformen gemäß **Fig. 9** und **10** jedoch nicht das hier gar nicht vorhandene Trägervlies 9, sondern vielmehr die jeweilige Deckschicht 3 bzw. 4 um die seitlichen Kanten des Produkts herumgefaltet. In dieser Beziehung entsprechen die Ausführungsformen gemäß **Fig. 9** und **10** im Wesentlichen der schematischen Darstellung gemäß **Fig. 5.**

Der Unterschied zwischen der Ausführungsform gemäß **Fig. 9** und der Ausführungsform gemäß **Fig. 10** besteht darin, dass bei der Vliesanordnung gemäß **Fig. 9** die Deckschicht durch die hier etwas stärkere, hydrophile Meltblown-Lage 3 gebildet wird, die an der Oberfläche zur Verbesserung der Oberflächenstabilität zusätzlich thermisch gebunden sein kann. Hingegen besteht bei der Vliesanordnung gemäß **Fig. 10** die Deckschicht lediglich aus dem hydrophilen Spinnvlies 4, was zu einer ganz besonders geringen Dicke eines so aufgebauten Hygieneprodukts führt.

Rückseitig sind die Vliesanordnungen gemäß **Fig. 9** und **10** wiederum mit einem punkt- bzw. streifenförmigen Haftschmelzkleberauftrag 12 versehen, der dafür sorgt, dass das jeweilige Hygieneprodukt beim Tragen nicht verrutschen kann. Zum Schutz des Haftschmelzklebers 12 ist auf der außenliegenden Seite des Haftschmelzkleber-Auftrags 12 wieder eine Lage Trennpapier 13, beispielsweise Silikonpapier, angeordnet.

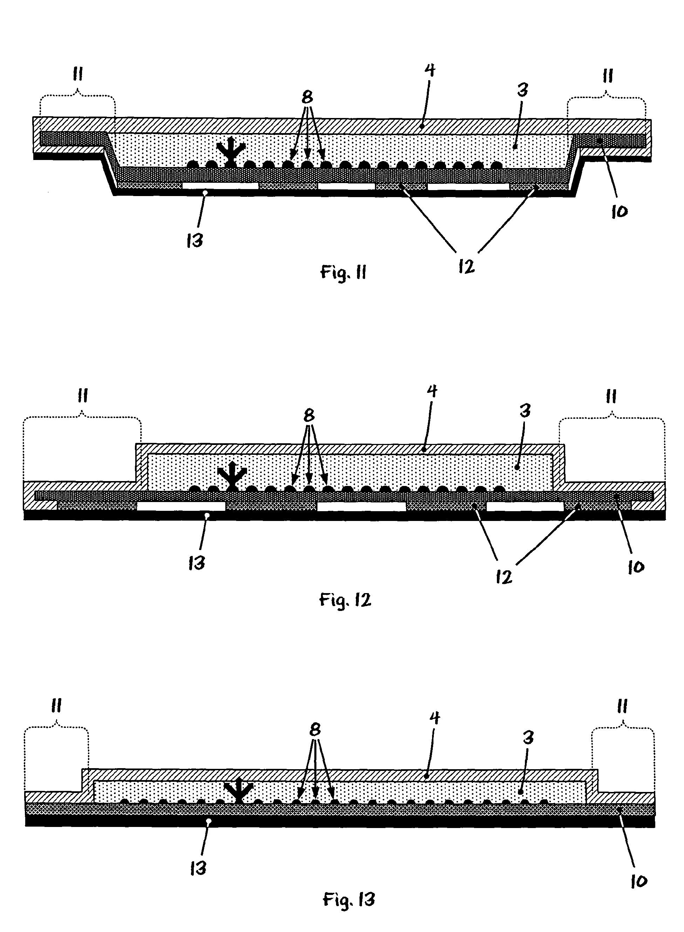

Bei den Vliesanordnungen gemäß **Fig. 11** bzw. **12** ist wiederum die Spunbound-Schicht 4 der SM-Decklage 3, 4 um die seitlichen Kanten des Produkts herumgefaltet, um einen guten Zusammenhalt der verschiedenen Lagen zu erreichen und zudem für wirksamen Auslaufschutz an den Kanten des Produkts zu sorgen. Letzterem dient ebenfalls der selektive Auftrag des Superabsorberpolymers 8 lediglich im mittleren Flächenbereich der Vliesanordnung sowie der seitliche Einzug 11 der Meltblown-Erfassungsschicht 3.

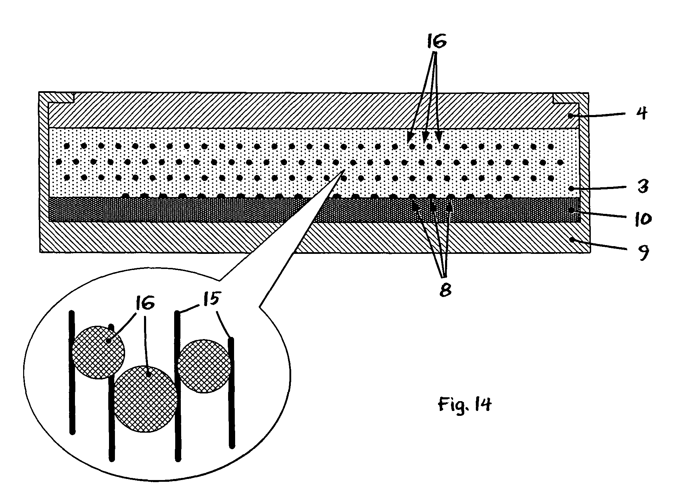

Bei der Vliesanordnung gemäß **Fig. 13** sind zur weiteren Vereinfachung des Aufbaus auch noch die Punkte bzw. Streifen des Haftschmelzkleber-Auftrags 12 gemäß **Fig. 9** und **10** entfallen. Die Aufgabe der Haftschmelz-Klebestreifen 12 wird bei der Ausführungsform gemäß **Fig. 13** ebenfalls durch ein und denselben Hotmelt-Auftrag 10 erfüllt, der gleichzeitig auch noch die Trägerschicht bildet und die Verankerung des Superabsorberpolymers 8 in der Vliesanordnung übernimmt. Zu diesem Zweck besteht die Hotmelt-Schicht 10 bei der Ausführungsform gemäß **Fig. 13** aus einem Kontakt-Hotmelt 10, dessen Oberfläche auch im abgekühlten Zustand eine gewisse Klebrigkeit aufweist. Die rückwärtige Oberfläche der Kontakt-Hotmeltschicht 10 wird auch bei der Ausführungsform gemäß **Fig. 13** durch ein Trennpapier abgedeckt, das vor dem Gebrauch eines dementsprechenden Hygieneprodukts abgezogen wird.

Die Vliesanordnung gemäß **Fig. 13** erfüllt somit einerseits höchste Ansprüche an spezifische Feuchtigkeitsaufnahme sowie Auslaufsicherheit bei geringem Gewicht und äußerst geringer Dicke, ist anderseits jedoch nahezu konkurrenzlos günstig herstellbar, da die Vliesanordnung gemäß **Fig. 13** nur aus einem Minimum an verschiedenen Lagen besteht.

Die Vliesanordnung gemäß **Fig. 14** schließlich entspricht im wesentlichen Schichtenaufbau der Vliesanordnung gemäß **Fig. 2**. Allerdings besitzt die Vliesanordnung gemäß **Fig. 14** zusätzlich zu dem auf der Hotmelt-Schicht 10 angeordneten Superabsorberpolymer zusätzlich Superabsorberpolymer-Partikel 16, die im Bereich der Meltblown-Lage 3 der Vliesanordnung angeordnet sind. Dabei sind die Superabsorberpolymer-Partikel 16 an die Fasern 15 der Meltblown-Lage 3 angelagert bzw. haften an den Fasern 15 der Meltblown-Lage 3. Auf diese Weise lässt sich die Absorptionskapazität und Speicherfähigkeit der Vliesanordnung bei gleichzeitig unverändert geringer Dicke der Vliesanordnung weiter verbessern. Auch die Auslaufsicherheit bei Druckbeanspruchung wird durch die Anlagerung der SAP-Partikel an die Mikrofaser des Meltblown 3 noch weiter erhöht.

Im Ergebnis wird damit deutlich, dass sich dank der erfindungsgemäßen Vliesanordnungen und dank der erfindungsgemäßen Verfahren zur Herstellung von Vliesanordnungen wichtige Eigenschaften wie Dicke, Wasserdampfdurchlässigkeit, Flüssigkeitsdichtigkeit, Flüssigkeitsaufnahme, mechanische Beanspruchbarkeit und Tragekomfort von hochabsorptionsfähigen Hygieneartikeln, insbesondere von Slipeinlagen oder Windeln, entscheidend verbessern lassen. Dabei werden die Herstellungskosten solcher Vliesanordnungen dank der Erfindung trotz verbesserter Produkteigenschaften nicht erhöht, sondern können, insbesondere dank der erfindungsgemäßen Verfahren, teilweise sogar erheblich reduziert werden.

## Patentansprüche

1. Mehrlagige Vliesanordnung, insbesondere für absorptive Hygieneartikel, die Vliesanordnung enthaltend Superabsorberpolymer (8) und umfassend eine Trägerlage, beispielsweise ein Trägervlies (9), sowie eine Decklage (3, 4),
**dadurch gekennzeichnet,**
**dass** die Vliesanordnung einen Film aus Heißschmelzkleber (10) umfasst, wobei partikelförmiges Superabsorberpolymer (8) bereichsweise auf der Oberfläche des Heißschmelzklebers (10) angeordnet ist.

2. Vliesanordnung nach Anspruch 1,
**dadurch gekennzeichnet,**
**dass** die Trägerlage (9) ein PP-, PET- oder PE- Spinn- oder Nadelvlies, ein wasserverfestigtes Vlies oder ein Mikrofaservlies ist.

3. Vliesanordnung nach Anspruch 1 oder 2,
**dadurch gekennzeichnet,**
**dass** die Trägerlage (9) ein Spinnvlies aus PP, HDPE oder LDPE mit einem Flächengewicht von 8 bis 80 g/m² ist.

4. Mehrlagige Vliesanordnung, insbesondere für absorptive Hygieneartikel, die Vliesanordnung umfassend eine Trägerlage sowie eine Decklage (3, 4),
**dadurch gekennzeichnet,**
**dass** die Trägerlage durch einen Film aus Heißschmelzkleber (10) gebildet ist.

5. Vliesanordnung nach Anspruch 4,
**dadurch gekennzeichnet,**
**dass** der Heißschmelzkleber (10) vom Typ Kontakt-Hotmelt ist.

6. Vliesanordnung nach Anspruch 4 oder 5,
**dadurch gekennzeichnet,**
**dass** die Vliesanordnung Superabsorberpolymer (8) enthält.

7. Vliesanordnung nach einem der Ansprüche 4 bis 6,
**dadurch gekennzeichnet,**
**dass** das Superabsorberpolymer (8) partikelförmig auf der Oberfläche des Heißschmelzklebers (10) angeordnet ist.

8. Vliesanordnung nach einem der Ansprüche 4 bis 7,
**dadurch gekennzeichnet,**
**dass** das Superabsorberpolymer (8) bereichsweise auf der Oberfläche des Heißschmelzklebers (10) angeordnet ist.

9. Vliesanordnung nach einem der Ansprüche 1 bis 8,
**dadurch gekennzeichnet,**
**dass** Trägerlage (9) und/oder Heißschmelzkleber (10) hydrophob sind.

10. Vliesanordnung nach einem der Ansprüche 1 bis 9,
**dadurch gekennzeichnet,**
**dass** der Heißschmelzkleber (10) vom Typ EVA, PA, PVAL oder PUR ist.

11. Vliesanordnung nach einem der Ansprüche 1 bis 10,
**dadurch gekennzeichnet,**
**dass** der Heißschmelzkleber (10) dampfdicht ist.

12. Vliesanordnung nach einem der Ansprüche 1 bis 10,
**dadurch gekennzeichnet,**
**dass** der Heißschmelzkleber (10) diffusionsoffen ist.

13. Vliesanordnung nach einem der Ansprüche 1 bis 12,
**dadurch gekennzeichnet,**
**dass** der Heißschmelzkleber (10) zumindest einen hydrophilen Bestandteil aufweist.

14. Vliesanordnung nach einem der Ansprüche 1 bis 13,
**dadurch gekennzeichnet,**
**dass** das Auftragsgewicht des Heißschmelzklebers (10) zwischen 5 bis 50 g/m² liegt.

15. Vliesanordnung nach einem der Ansprüche 1 bis 14,
**dadurch gekennzeichnet,**
**dass** die Decklage (3, 4) ein hydrophiles Mikrofaservlies (3) umfasst.

16. Vliesanordnung nach einem der Ansprüche 1 bis 15,
**dadurch gekennzeichnet,**
**dass** die Decklage (3, 4) als SM-Verbund aus hydrophilem Spinnvlies (4) mit einem bevorzugten Flächengewicht von 8 bis 80g/m² und aus hydrophilem Meltblown (3) mit einem bevorzugten Flächengewicht von 10 bis 100 g/m² ausgebildet ist.

17. Vliesanordnung nach einem der Ansprüche 1 bis 16,
**dadurch gekennzeichnet,**
**dass** die Decklage (3, 4) durch öl-, wachs- oder fetthaltige Avivagen hautfreundlich ausgerüstet ist.

18. Vliesanordnung nach einem der Ansprüche 1 bis 17,
**dadurch gekennzeichnet,**
**dass** die Außenkanten der Trägerlage (9) oder der Decklage (3, 4) zumindest bereichsweise einen Randumschlag um die Außenkanten der Vliesanordnung bilden.

19. Vliesanordnung nach einem der Ansprüche 1 bis 18,
**dadurch gekennzeichnet,**
**dass** das Superabsorberpolymer (8) zumindest teilweise in Form eines Naturprodukts vorliegt, insbesondere in Form der gemahlenen Frucht des Johannisbrotbaumes.

20. Vliesanordnung nach einem der Ansprüche 1 bis 19,
**dadurch gekennzeichnet,**
**dass** dem Superabsorberpolymer (8) Aktivkohle zugesetzt ist.

21. Vliesanordnung nach einem der Ansprüche 1 bis 20,
**dadurch gekennzeichnet,**
**dass** dem Superabsorberpolymer (8) verkokter, pulverisierter Bambus zugesetzt ist.

22. Vliesanordnung nach einem der Ansprüche 1 bis 21,
**dadurch gekennzeichnet,**
**dass** das hydrophile Mikrofaservlies (3) Superabsorberpolymer (16) umfasst.

23. Vliesanordnung nach Anspruch 22,
**dadurch gekennzeichnet,**
**dass** Superabsorberpolymer-Partikel (16) an die Fasern (15) des hydrophilen Mikrofaservlieses (3) angelagert sind (bzw. anhaften).

24. Verfahren zur Herstellung einer Vliesanordnung mit den Verfahrensschritten:
a) Aufbringen eines Hotmeltfilms (10) auf eine Trägerlage (9);
a') Selektiv bereichsweises Aufbringen von Superabsorberpolymer-Partikeln (8) auf den noch schmelzklebrigen Hotmeltfilm (10);
b) Aufbringen einer Decklage (3, 4) auf Hotmeltfilm (10) und Superabsorberpolymer-Partikel (8); und
c) Zuschnitt der Vliesanordnung und Verbindung der Lagen.

25. Verfahren zur Herstellung einer Vliesanordnung mit den Verfahrensschritten:
a) Aufbringen zumindest einer Lage Hotmeltfilm (10) auf ein Trennpapier (13);
b) Aufbringen einer Decklage (3, 4) auf den Hotmeltfilm (10); und
c) Zuschnitt der Vliesanordnung und Verbindung der Lagen.

26. Verfahren nach Anspruch 25,
**dadurch gekennzeichnet,**
**dass** Superabsorberpolymer (8) in die Vliesanordnung eingebracht wird oder das Ausgangsmaterial der Decklage (3, 4) Superabsorberpolymer (8) enthält.

27. Verfahren nach Anspruch 25 oder 26,
**dadurch gekennzeichnet,**
**dass** in einem weiteren Verfahrensschritt a') Superabsorberpolymer (8) auf den noch schmelzklebrigen Hotmeltfilm (10) aufgebracht wird.

28. Verfahren nach einem der Ansprüche 25 bis 27,
**dadurch gekennzeichnet,**
**dass** das Superabsorberpolymer (8) partikelförmig ist.

29. Verfahren nach einem der Ansprüche 25 bis 28,
**dadurch gekennzeichnet,**
**dass** das Superabsorberpolymer (8) selektiv bereichsweise auf dem Hotmeltfilm (10) angeordnet wird.

30. Verfahren nach einem der Ansprüche 24 oder 27 bis 29,
**dadurch gekennzeichnet,**
**dass** das Aufbringen der Superabsorberpolymer-Partikel (8) im Verfahrensschritt a') durch eine Druckwalze, insbesondere eine Gravurwalze erfolgt.

31. Verfahren nach einem der Ansprüche 24 oder 27 bis 29,
**dadurch gekennzeichnet,**
**dass** das Aufbringen der Superabsorberpolymer-Partikel (8) im Verfahrensschritt a') durch elektrostatisches Sprühen erfolgt.

32. Verfahren nach einem der Ansprüche 24 bis 31,
**dadurch gekennzeichnet,**
**dass** die Decklage (3, 4) im Verfahrensschritt c) ein hydrophiler SM-Verbund aus Spinnvlies (4) und Meltblown (3) ist.

33. Verfahren nach einem der Ansprüche 24 bis 32,
**dadurch gekennzeichnet,**
**dass** zwischen den Verfahrensschritten b) und c) oder nach Verfahrensschritt c) in einem weiteren Verfahrensschritt c') überstehende Randbereiche der Trägerlage (9) oder der Decklage (3, 4), einen Randumschlag bildend, um korrespondierende Randbereiche der Vliesanordnung herumgefaltet werden.

34. Verfahren nach einem der Ansprüche 24 bis 33,
**dadurch gekennzeichnet,**
**dass** die Verbindung der Lagen im Verfahrensschritt c) durch Kalandrierung erfolgt.

35. Verfahren nach einem der Ansprüche 24 bis 34,
**dadurch gekennzeichnet,**
**dass** in weiteren Verfahrensschritten d) und e) auf der Rückseite der Trägerschicht ein Auftrag von Heißschmelz-Kontaktkleber (12) sowie von Trennpapier (13), beispielsweise Silikonpapier, erfolgt.

36. Verfahren nach einem der Ansprüche 24 bis 35,
**dadurch gekennzeichnet,**
**dass** die Verfahrensschritte im getakteten oder kontinuierlichen Inline-Verfahren durchgeführt werden.
